# EUROPEAN PATENT APPLICATION

(11) **EP 3 376 198 A1**
(43) Date of publication of application: **19.09.2018**
(21) Application number: 18161663.2
(22) Date of filing: 14.03.2018
(51) Int. Cl.: G01M 15/10, G01M 17/007

(54) **VEHICLE-MOUNTED EXHAUST GAS ANALYZER, EXHAUST GAS ANALYSIS SYSTEM, INFORMATION PROCESSING DEVICE, PROGRAM FOR EXHAUST GAS ANALYSIS SYSTEM, AND EXHAUST GAS ANALYSIS METHOD**

(30) Priority: 15.03.2017 JP 2017050047
(71) Applicant: Horiba, Ltd., Kyoto-shi, Kyoto 601-8510 (JP)
(72) Inventor: FURUKAWA, Kazuki, Kyoto, 601-8510 (JP); IKEDA, Hiroyuki, Kyoto, 601-8510 (JP)
(74) Representative: Müller Hoffmann & Partner

(57) **Abstract**

In order to make it possible to perform exhaust gas analysis by a vehicle-mounted exhaust gas analyzer 10 and the analysis, evaluation, and the like of results of the analysis in consideration of a running state of a surrounding vehicle V2 during the exhaust gas analysis, the vehicle-mounted exhaust gas analyzer 10 mounted in a test vehicle VI running on a road is adapted to include: an exhaust gas data acquisition part 21 adapted to acquire exhaust gas data on exhaust gas discharged from the test vehicle during the run of the test vehicle VI; and a surrounding vehicle information acquisition part 23 adapted to, from surrounding vehicle sensing means 30 mounted in the test vehicle VI, acquire surrounding vehicle information that is information on a vehicle running around the test vehicle VI.

## Description

### Technical Field

The present invention relates to a vehicle-mounted exhaust gas analyzer, an exhaust gas analysis system, an information processing device, a program for the exhaust gas analysis system, and an exhaust gas analysis method.

### Background Art

As a conventional exhaust gas analysis system, as disclosed in Patent Literature 1, there is one configured to include a vehicle-mounted exhaust gas analyzer mounted in a test vehicle and analyze exhaust gas discharged from an internal combustion engine during the run of the test vehicle by the vehicle-mounted exhaust gas analyzer.

Meanwhile, when during the run of the test vehicle on, for example, a highway, a large vehicle runs in front, the air resistance of the test vehicle is reduced due to the effect of the vehicle in front. Note that such a phenomenon is caused not only by a vehicle in front but also by a vehicle running on left or right of or in back of the test vehicle.

This indicates that depending on the presence or absence of a surrounding vehicle running around the test vehicle, the inter-vehicle distance between the test vehicle and a surrounding vehicle, the size of a surrounding vehicle, or the like, analysis results obtained by the vehicle-mounted exhaust gas analyzer may significantly differ. In such a case, there occurs a problem of the poor reproducibility of analysis results in a test such as a repeat test.

However, there has been no idea of taking account of the effect of a surrounding vehicle during exhaust gas analysis by a vehicle-mounted exhaust gas analyzer in the exhaust gas analysis and in the analysis, evaluation, and the like of results of the exhaust gas analysis.

### Citation List

### Patent Literatures

Patent Literature 1: Japanese Unexamined Patent Application Publication No. 2005-178692

### Summary of Invention

### Technical Problem

Therefore, the present invention is made on the basis of the non-conventional idea in order to solve the above-described problem, and a main object thereof is to make it possible to, in exhaust gas analysis using a vehicle-mounted exhaust gas analyzer, take account of the effect of a surrounding vehicle during the exhaust gas analysis.

### Solution to Problem

That is, a vehicle-mounted exhaust gas analyzer according to the present invention is one mounted in a test vehicle running on a road, and the vehicle-mounted exhaust gas analyzer includes: an exhaust gas data acquisition part adapted to acquire exhaust gas data on exhaust gas discharged from the test vehicle during the run of the test vehicle; and a surrounding vehicle information acquisition part adapted to, from surrounding vehicle sensing means mounted in the test vehicle, acquire surrounding vehicle information that is information on a vehicle running around the test vehicle.

In the vehicle-mounted exhaust gas analyzer configured as described above, since the surrounding vehicle information acquisition part can acquire the surrounding vehicle information by the surrounding vehicle sensing means during the acquisition of the exhaust gas data by the exhaust gas data acquisition part, the exhaust gas analysis and the analysis, evaluation, and the like of results of the analysis can be performed in consideration of the effect of the surrounding vehicle during the acquisition of the exhaust gas data.

In order to make it possible to compare the exhaust gas data and the surrounding vehicle information during the acquisition of the exhaust gas with each other, it is preferable that the vehicle-mounted exhaust gas analyzer includes an on-road test data storage part adapted to store the exhaust gas data and the surrounding vehicle information in relation to each other.

In order to make it possible to perform the exhaust gas analysis and the analysis, evaluation, and the like of the results of the analysis in consideration of at least the inter-vehicle distance between the test vehicle and the surrounding vehicle during the acquisition of the exhaust gas data, it is preferable that the surrounding vehicle information includes inter-vehicle distance information indicating the inter-vehicle distance between the test vehicle and the surrounding vehicle during the acquisition of the exhaust gas data by the exhaust gas data acquisition part.

Specific embodiments include a configuration in which at least one of a millimeter wave radar or an imaging device is mounted in the test vehicle as the surrounding vehicle sensing means.

The effect of a vehicle in front on air resistance applied to the test vehicle depends not only on the inter-vehicle distance between the vehicle in front and the test vehicle but also on the size of the vehicle in front. Accordingly, in order to make it possible to more accurately take account of the effect, it is preferable that the surrounding vehicle information acquisition part acquires vehicle area information that is information obtained with use of imaged data by the imaging device and indicates the back surface area of the vehicle in front imaged during the exhaust gas analysis.

In order to objectively determine the effectiveness of the exhaust gas data on the basis of the effect of the surrounding vehicle, it is preferable that the vehicle-mounted exhaust gas analyzer includes a test effectiveness determination part adapted to, on the basis of at least the inter-vehicle distance information, determine the effectiveness of the exhaust gas data in an on-road running test of the test vehicle.

Specific embodiments of the test effectiveness determination part include a configuration in which the test effectiveness determination part measures time periods during which the inter-vehicle distance indicated by the inter-vehicle distance information exceeds a predetermined threshold value, and when the time periods exceed a predetermined time period continuously or in total, determines that the exhaust gas data is effective data.

It is preferable that the vehicle-mounted exhaust gas analyzer includes a reporting part adapted to, when the test effectiveness determination part determines that the exhaust gas data is effective data, report the result of the determination during the exhaust gas analysis.

Such a configuration allows a driver in the test vehicle to know the acquisition of the effective exhaust gas data during a run, and to be prevented from spending wasted running time by determining whether the run should be continued.

It is preferable that the vehicle-mounted exhaust gas analyzer includes a display part adapted to display the total of the time periods or distances during which the inter-vehicle distance indicated by the inter-vehicle distance information exceeds the predetermined threshold value, or the ratio of the total of the time periods or the distances to the total run period or distance of the test vehicle.

Such a configuration makes it possible to, while running, adjust the time periods or distances during which the inter-vehicle distance exceeds the predetermined threshold value, and improve the reproducibility of the exhaust gas data.

An information processing device according to the present invention is one used together with a vehicle-mounted exhaust gas analyzer mounted in a test vehicle running on a road, and the information processing device includes: an exhaust gas data acquisition part adapted to acquire exhaust gas data on exhaust gas discharged from the test vehicle during the run of the test vehicle; and a surrounding vehicle information acquisition part adapted to, from surrounding vehicle sensing means mounted in the test vehicle, acquire surrounding vehicle information that is information on a vehicle running around the test vehicle.

Also, an exhaust gas analysis system according to the present invention includes: the vehicle-mounted exhaust gas analyzer; and the above-described information processing device.

Further, a program for an exhaust gas analysis system according to the present invention is a program used for an exhaust gas analysis system including a vehicle-mounted exhaust gas analyzer mounted in a test vehicle running on a road, and the program instructs a computer to fulfill functions as: an exhaust gas data acquisition part adapted to acquire exhaust gas data on exhaust gas discharged from the test vehicle during the run of the test vehicle; and a surrounding vehicle information acquisition part adapted to, from surrounding vehicle sensing means mounted in the test vehicle, acquire surrounding vehicle information that is information on a vehicle running around the test vehicle.

In addition, an exhaust gas analysis method according to the present invention is one including steps of: acquiring exhaust gas data on exhaust gas discharged from the test vehicle during the run of the test vehicle with use of a vehicle-mounted exhaust gas analyzer mounted in a test vehicle; and acquiring surrounding vehicle information that is information on a vehicle running around the test vehicle from surrounding vehicle sensing means mounted in the test vehicle.

Such an information processing device, an exhaust gas analysis system, a program for the exhaust gas analysis system, and an exhaust gas analysis method can produce the same working effect as that of the above-described vehicle-mounted exhaust gas analyzer,.

### Advantageous Effects of Invention

According to the present invention configured as described above, the exhaust gas analysis by the vehicle-mounted exhaust gas analyzer, and the analysis, evaluation, and the like of results of the analysis can be performed in consideration of a running state of a surrounding vehicle during the exhaust gas analysis.

### Brief Description of Drawings

FIG. 1 is a schematic diagram illustrating the configuration of an exhaust gas analysis system of the present embodiment;
FIG. 2 is a functional block diagram illustrating functions of an information processing device of the same embodiment;
FIG. 3 is a flowchart illustrating the operation of the exhaust gas analysis system of the same embodiment;
FIG. 4 is a schematic diagram illustrating the configuration of an exhaust gas analysis system of a variation;
FIG. 5 is a functional block diagram illustrating functions of an information processing device of another variation;
FIG. 6 is a functional block diagram illustrating functions of an information processing device of still another variation;
FIG. 7 is a schematic diagram illustrating the configuration of an exhaust gas analysis system of yet another variation; and
FIG. 8 is a functional block diagram illustrating functions of a vehicle-mounted exhaust gas analyzer of still yet another variation. Description of Embodiments

In the following, one embodiment of the exhaust gas analysis system according to the present invention will be described with reference to the drawings.

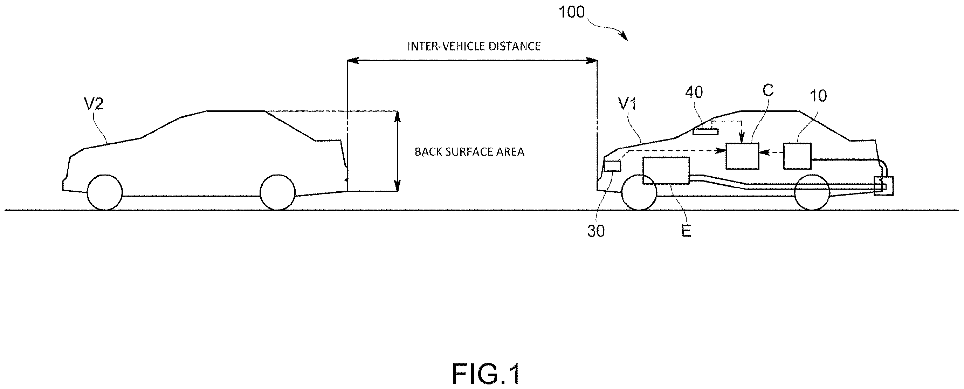

An exhaust gas analysis system 100 of the present embodiment is one for analyzing exhaust gas discharged from an internal combustion engine E of a test vehicle V1 running on a road, and specifically, as illustrated in FIG. 1, includes: a vehicle-mounted exhaust gas analyzer 10 mounted in the test vehicle V1; and an information processing device C adapted to acquire exhaust gas data indicating analysis results obtained by the vehicle-mounted exhaust gas analyzer 10.

The vehicle-mounted exhaust gas analyzer 10 is one adapted to be introduced with the exhaust gas discharged from the internal combustion engine E through, for example, a hot hose, and analyze a component in the exhaust gas, such as carbon monoxide (CO), carbon dioxide (CO₂), nitrogen oxide (NO_{X}), or total hydrocarbon (THC).

Specifically, as the vehicle-mounted exhaust gas analyzer 10, for example, one adapted to analyze the concentration of carbon oxide or carbon dioxide in the exhaust gas by a non-dispersive infrared absorption (NDIR) method, one adapted to analyze the concentration of nitrogen oxide in the exhaust gas by a chemiluminescence method or a non-dispersive ultraviolet analysis (NDUV) method, one adapted to analyze the concentration of total hydrocarbon in the exhaust gas by a hydrogen flame ionization (FID) method, one adapted to collect particulate matter (PM) contained in the exhaust gas using a filter to measure the weight of it, one adapted to count the particulate matter in the exhaust gas using a condensed particle counter (CPC), one using multiple methods among them, or the like can be cited. Note that the analysis herein is a concept including detecting the presence or absence of each component, measuring the concentration of each component, and the like.

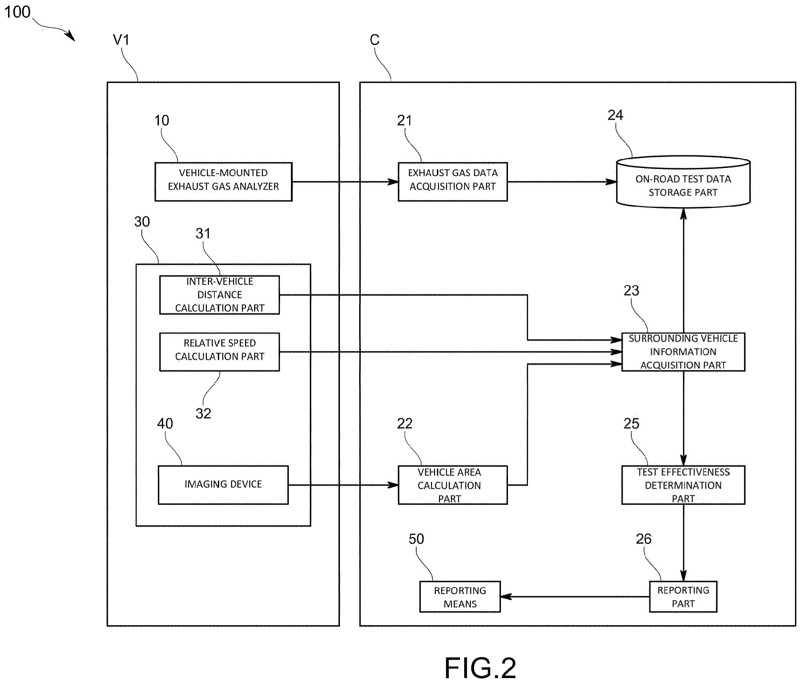

The information processing device C is a computer including a CPU, a memory, AC/DC converters, input means, and other such components, and configured to fulfill at least a function as an exhaust gas data acquisition part 21 as illustrated in FIG. 2 by executing various programs stored in the memory by the CPU.

The exhaust gas acquisition part 21 is one adapted to acquire the exhaust gas data obtained by the above-described vehicle-mounted exhaust gas analyzer 10, and specifically, may be configured to acquire the exhaust gas data by wire or wireless or via an external memory.

As illustrated in FIG. 1, the test vehicle V1 of the present embodiment is mounted with at least surrounding vehicle sensing means 30 adapted to sense a surrounding vehicle V2 running around the test vehicle V1. Note that the surrounding vehicle sensing means 30 may be one mounted in the test vehicle V1 as a component of the exhaust gas analysis system 100 or one preliminarily mounted in the test vehicle V1 at the time of manufacturing of the test vehicle V1 or on another occasion not as a component of the exhaust analysis system 100.

The surrounding vehicle sensing means 30 is one adapted to sense an object around the test vehicle V1 by transmitting a survey wave outward of the test vehicle V1, and receiving a reflected wave resulting from the reflection of the survey wave by the object. The surrounding vehicle sensing means 30 herein is a millimeter wave radar that is provided on the front side of the test vehicle V1, specifically installed inside or outside the vehicle, in order to sense at least the vehicle in front V2 running in front of the test vehicle V1.

The surrounding vehicle sensing means 30 of the present embodiment is configured to acquire, as sensing data, a time from transmitting the survey wave to receiving the reflected wave, and as illustrated in FIG. 2, includes a function as an inter-vehicle distance calculation part 31 adapted to calculate the inter-vehicle distance between the test vehicle V1 and the surrounding vehicle V2 (in this case, the vehicle in front V2) on the basis of the time.

In addition, the surrounding vehicle sensing means 30 is configured to acquire, as the sensing data, a frequency difference occurring between the survey wave and the reflected wave, and as illustrated in FIG. 2, further includes a function as a relative speed calculation part 32 adapted to calculate the relative speed of the surrounding vehicle V2 (in this case, the vehicle in front V2) to the test vehicle V1, i.e., the difference in vehicle speed between the test vehicle V1 and the surrounding vehicle V1 on the basis of the frequency difference. Note that the relative speed calculation part 32 may be configured to calculate the relative speed from the time change rate of the inter-vehicle distance calculated by the inter-vehicle distance calculation part 31.

In the present embodiment, the test vehicle V1 is further mounted with, as the surrounding vehicle sensing means 30, an imaging device 40 adapted to image the surrounding vehicle V2 running around the test vehicle V1.

The imaging device 40 is one adapted to image the outside of the test vehicle V1. The imaging device 40 herein is provided on the front side of the test vehicle V1 in order to image at least the vehicle V2 in front running in front of the test vehicle V1, and specifically a camera installed inside or outside the test vehicle V1. Note that the imaging device 40 is one constituting a drive recorder preliminarily mounted in the test vehicle V1.

In addition, as illustrated in FIG. 2, the information processing device C of the present embodiment is configured to further fulfill functions as a vehicle area calculation part 22, a surrounding vehicle information acquisition part 23, an on-road test data storage part 24, a test effectiveness determination part 25, and a reporting part 26 by executing the above-described various programs stored in the memory by the CPU.

The respective parts will be described below.

The vehicle area calculation part 22 is one adapted to acquire imaged data obtained by the imaging device 40, as well as to use the imaged data to calculate the back surface area of the vehicle in front V2 imaged during exhaust gas analysis. Note that the back surface area herein refers to the area of a viewable part when viewing the vehicle in front V2 from the back, and includes at least the area of a part that may affect air resistance applied to the test vehicle V1 running in back.

Specific configurations of the vehicle area calculation part 22 include one adapted to recognize a license plate by some means such as performing image analysis of the imaged data and calculate the above-described back surface area on the basis of the imaged area of the license plate. More specifically, for example, by preliminarily store the actual area of a license plate in the memory, the back surface area can be calculated from the ratio between the imaged area of the license plate and the actual area. For example, when the actual area of a license plate is denoted by S1, the imaged area of the imaged license plate by S2, and the imaged back surface area of the vehicle in front V2 by S3, the actual back surface area S4 of the vehicle in front V2 can be calculated as S4 = S1 × S3 / S2.

Note that the above-described back surface area can also be calculated using, in addition to the imaged area of the license plate, the imaged length of the license plate (such as horizontal length, vertical length, or separation distance between multiple predetermined positions of bolts or the like). For example, when the actual horizontal length of a license plate is denoted by L1, the imaged horizontal length of the imaged license plate by L2, and the imaged back surface area of the vehicle in front V2 by S3, the actual back surface area S4 of the vehicle in front V2 can be calculated as S4 = (L1)² × S3 / (L2)².

The surrounding vehicle information acquisition part 23 is one adapted to acquire surrounding vehicle information indicating the relationship between the test vehicle V1 and the surrounding vehicle V2, and the surrounding vehicle information includes information on the surrounding vehicle V2 that may affect the air resistance applied to the test vehicle V1.

Specifically, the surrounding vehicle information acquisition part 23 acquires the surrounding vehicle information including at least inter-vehicle distance information indicating the inter-vehicle distance calculated by the inter-vehicle distance calculation part 31.

The surrounding vehicle information of the present embodiment also includes, in addition to the inter-vehicle information, relative speed information indicating the relative speed calculated by the relative speed calculation part 32 and further back surface area information indicating the back surface area of the vehicle in front V2 calculated by the vehicle area calculation part 22.

The on-road test data storage part 24 is one that is set in a predetermined area of the memory and adapted to store the exhaust gas data acquired by the exhaust gas data acquisition part 21 and at least the inter-vehicle distance information acquired by the surrounding vehicle information acquisition part 23 in relation to each other.

Specifically, the on-road test data storage part 24 chronologically stores the exhaust gas data and the inter-vehicle distance information, and stores the exhaust gas data and the inter-vehicle distance information in relation to each other such that the time of analysis by the vehicle-mounted exhaust gas analyzer 10 and the running time at which the inter-vehicle distance between the test vehicle V1 and the vehicle in front V2 is equal to the distance indicated by the inter-vehicle distance information coincide with each other.

In the present embodiment, the on-road test data storage part 24 further stores, in addition to the inter-vehicle distance information, the relative speed information and the back surface area information in relation to the exhaust gas data. Specifically, in the same manner as above, the on-road test data storage part 24 stores the relative speed information and the back surface area information in relation to the exhaust gas data such that the analysis time and the running time coincide with each other. In addition, the on-road test data storage part 24 may store the vehicle speed of the test vehicle V1 and pieces of sensing information from various sensors constituting an advanced driver assistance system (ADAS) in relation to the exhaust gas data.

Note that the above-described various pieces of information such as the exhaust gas data, inter-vehicle distance information, relative speed information, and back surface area information may be adapted to be respectively transmitted to different information processing devices (memories). In addition, these pieces of information are preferably transmitted in real time.

The test effectiveness determination part 25 is one adapted to determine the effectiveness of the exhaust gas data on the basis of at least the inter-vehicle distance information acquired by the surrounding vehicle information acquisition part 23.

Specific configurations of the test effectiveness determination part 25 include one adapted to measure time periods during which the inter-vehicle distance to the vehicle in front V2 during exhaust gas analysis exceeds a predetermined threshold value, and when the time periods exceed a predetermined lower limit time period continuously or in total, determine that exhaust gas data obtained during a relevant running test is effective data. In addition, the test effectiveness determination part 25 may be configured to determine that exhaust gas data obtained during time periods during which the inter-vehicle distance between the test vehicle V1 and the surrounding vehicle V2 fall below the predetermined threshold value is ineffective data.

Also, the test effectiveness determination part 25 may be configured to determine the effectiveness of exhaust gas data using, in addition to the inter-vehicle distance to the vehicle in front V2 during exhaust gas analysis, the relative speed to the vehicle in front V2 during the exhaust gas analysis and further the back surface area of the vehicle in front V2 during the exhaust gas analysis as criteria.

Note that in an opposite manner to the above configuration, the test effectiveness determination part 25 may be configured to determine that exhaust gas data is ineffective data. That is, the test effectiveness determination part 25 may be adapted to measure time periods during which the inter-vehicle distance to the vehicle in front V2 during exhaust gas analysis falls below the predetermined threshold value, and when the time periods exceed a predetermined upper limit time period continuously or in total, determine that exhaust gas data obtained during at least the time periods during which the inter-vehicle distance falls below the threshold value is ineffective data.

The reporting part 26 is one adapted to, when exhaust gas data is determined to be effective data by the test effectiveness determination part 25, reports the determination result during exhaust gas analysis, and specifically, outputs a reporting signal to reporting means 50 mounted in the information processing device C, such as a light emission part and/or a sound output part, to produce light, sound, and/or the like. In addition, the reporting part 26 may be one adapted to, on a display included in the information processing device C, display that exhaust gas data is effective data.

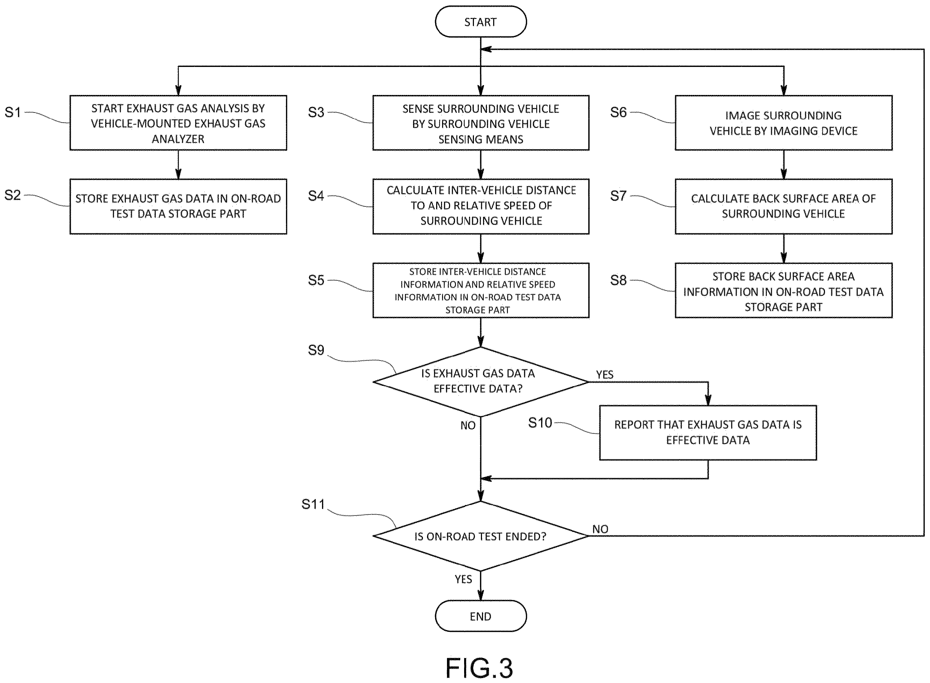

Next, the operation of the exhaust gas analysis system 100 of the present embodiment will be described with reference to a flowchart of FIG. 3.

First, when a running test using the test vehicle V1 is started, the vehicle-mounted exhaust gas analyzer 10 starts exhaust gas analysis, and exhaust gas data obtained by the vehicle-mounted exhaust gas analyzer 10 is successively outputted to the information processing device C (S1).

The outputted exhaust gas data is successively acquired by the exhaust gas data acquisition part 21 of the information processing device C, and also chronologically stored in the on-road test data storage part 24 (S2).

Meanwhile, the surrounding vehicle sensing means 30 senses the surrounding vehicle V2 during the acquisition of the exhaust gas data by the vehicle-mounted exhaust gas analyzer 10. Specifically, the millimeter wave radar as the surrounding vehicle sensing means 30 senses the vehicle in front V2 by transmitting the survey wave and receiving the survey wave reflected from the vehicle in front V2 (S3). During the sensing, the millimeter wave radar 30 calculates the inter-vehicle distance between the vehicle in front V2 and the test vehicle V1 during the analysis by the inter-vehicle distance calculation part 31 included therein as its function, as well as calculates the relative speed of the vehicle in front V2 during the analysis by the relative speed calculation part 32 (S4). The inter-vehicle distance information indicating the calculated inter-vehicle distance and the relative speed information indicating the calculated relative speed are successively outputted from the millimeter wave radar 30 to the information processing device C.

The outputted inter-vehicle distance information and relative speed information are successively acquired by the surrounding vehicle information acquisition part 23 of the information processing device C, and also chronologically stored in the on-road test data storage part 24 (S5).

In addition, in the present embodiment, the imaging device 40 images the vehicle in front V2 during the acquisition of the exhaust gas data by the vehicle-mounted exhaust gas analyzer 10 (S6). Specifically, the digital camera as the imaging device 40 images the vehicle in front V2 during the analysis, and the imaged data is successively outputted from the imaging device 40 to the information processing device C. The outputted imaged data is acquired by the vehicle area calculation part 22 of the information processing device C, and the vehicle area calculation part 22 calculates the back surface area of the vehicle in front V2 on the basis of the imaged data (S7).

The back surface area information indicating the calculated back surface area is acquired by the surrounding vehicle information acquisition part 23, and also chronologically stored in the on-road test data storage part 24 (S8).

In the present embodiment, the on-road test data storage part 24 stores the exhaust gas data and at least the inter-vehicle distance information in relation to each other, and the test effectiveness determination part 25 determines the effectiveness of the exhaust gas data using at least the inter-vehicle distance information stored in relation to the exhaust gas data (S9). A specific determination method for the effectiveness of the exhaust gas data is as described above.

When the test effectiveness determination part 25 determines that the exhaust gas data is effective data, the reporting part 26 reports the determination result (S10). In doing so, a driver can determine whether to end the on-road test or further continue the run.

Then, for example, an unillustrated test end determination part of the information processing device C determines whether a test end signal indicating that the test is to be ended is inputted (S11), and when the test end signal is inputted, the exhaust gas analysis and the like by the vehicle-mounted exhaust gas analyzer 10 are stopped and the on-road test is ended.

In the exhaust gas analysis system 100 according to the present embodiment configured as described above, since the surrounding vehicle information acquisition part 23 acquires the inter-vehicle distance information indicating the inter-vehicle distance between the test vehicle V1 and the vehicle in front V2 during exhaust gas analysis, the exhaust gas analysis by the vehicle-mounted exhaust gas analyzer 10 and the analysis, evaluation, and the like of results of the analysis can be performed in consideration of the effect of at least the inter-vehicle distance.

In addition, since the surrounding vehicle information acquisition part 23 further acquires the relative speed information indicating the relative speed of the vehicle in front V2 to the test vehicle V1 during exhaust gas analysis and the back surface area information indicating the back surface area of the vehicle in front V2 during the exhaust gas analysis, using these pieces of information allows the exhaust gas analysis and the like to be performed while more accurately taking account of, for example, the effect of the vehicle in front V2 on air resistance applied to the test vehicle V1.

Further, since the test effectiveness determination part 25 determines the effectiveness of exhaust gas data on the basis of the inter-vehicle distance and the like during exhaust gas analysis, the effectiveness of the exhaust gas data can be objectively determined.

In addition, since when the test effectiveness determination part 25 determines that exhaust gas data is effective data, the reporting part 26 reports the determination result, a driver in the test vehicle V1 can know during a run that the effective exhaust gas data has been obtained. This allows the driver to determine during the run whether the test run should be continued, and to be prevented from spending wasted running time.

Note that the present invention is not limited to the above-described embodiment.

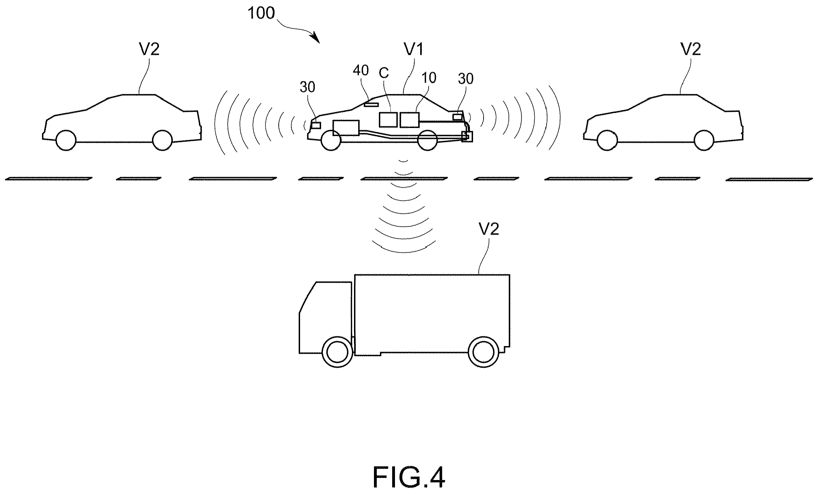

For example, in the above-described embodiment, described is the case where the surrounding vehicle sensing means 30 senses the vehicle in front V2. However, the present invention may be adapted to mount multiple vehicle sensing means 30 in the test vehicle V1, and as illustrated in FIG. 4, to sense surrounding vehicles V2 running in front and back of and on left or right of the test vehicle V1.

The same idea is also applicable to the imaging device 40. That is, the present invention may be adapted to mount multiple imaging devices 40 in the test vehicle V1 and image the surrounding vehicles V2 running in front and back of and on left or right of the test vehicle V1.

In addition, the inter-vehicle distance calculation part 31 may be configured to calculate not only the inter-vehicle distance to the vehicle in front but the inter-vehicle distances to the vehicles in back and on left or right; the relative speed calculation part 32 may be configured to calculate the relative speeds of the surrounding vehicles V2 to the test vehicle; and the vehicle area calculation part 22 may be configured to calculate the vehicle areas of the surrounding vehicles V2, such as the areas of front and side surfaces.

Such configurations make it possible to perform analysis by the vehicle-mounted exhaust gas analyzer 10 and the analysis, evaluation, and the like of results of the analysis in consideration of not only the effect of the vehicle in front V2 but the effect of the surrounding vehicles V2 running in back and on either side.

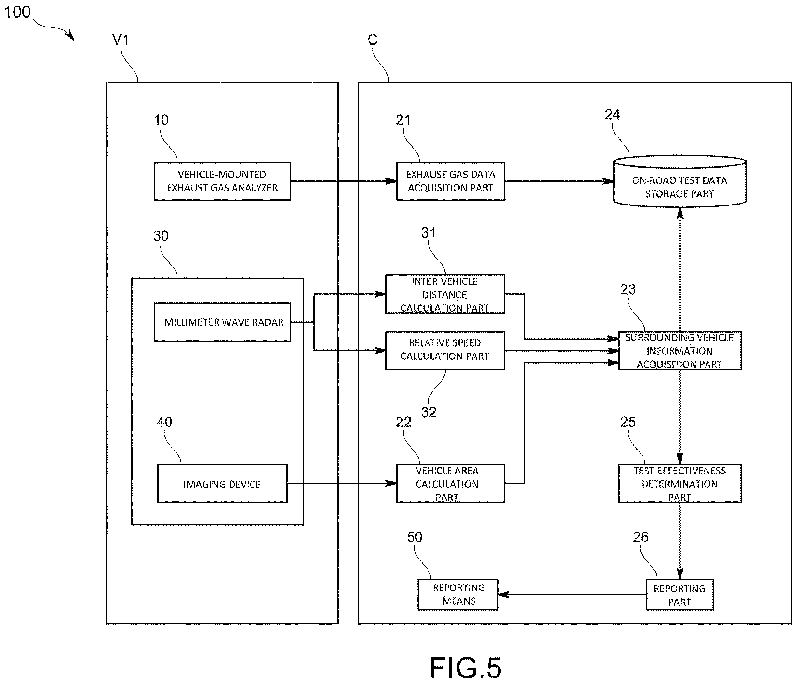

Further, in the above-described embodiment, described is the case where the surrounding vehicle sensing means 30 includes the functions as the inter-vehicle distance calculation part 31 and the relative speed calculation part 32. However, as illustrated in FIG. 5, one or both of the functions may be included in the information processing device C.

In addition, the surrounding vehicle sensing means 30 in the above-described embodiment includes both of the millimeter wave radar and the imaging device, but may be one including any one of them.

In the case where the surrounding vehicle sensing means 30 includes only the imaging device 40, as the inter-vehicle distance calculation part 31, a configuration adapted to calculate the inter-vehicle distance between the test vehicle V1 and the surrounding vehicle V2 during exhaust gas analysis by some means such as performing image analysis on imaged data obtained by the imaging device can be cited. The same configuration is also applicable to the relative speed calculation part 32. That is, as the relative speed calculation part 32, a configuration adapted to calculate the relative speed of the surrounding vehicle V2 to the test vehicle V1 during exhaust gas analysis by some means such as performing image analysis on imaged data obtained by the imaging device 40 can be cited.

In addition, the surrounding vehicle sensing means 30 may sense not only the surrounding vehicle V2 but also a surrounding object around the test vehicle V1, such as a wall or a traffic signal. The same configuration is also applicable to the imaging device 40, and the imaging device 40 may image a surrounding object around the test vehicle V1. Further, the imaging device 40 may image the condition of a road on which the test vehicle V1 is running, such as roughness or a curve.

Such configurations make it possible to perform exhaust gas analysis by the vehicle-mounted exhaust gas analyzer 10 and the analysis, evaluation, and the like of results of the analysis in consideration of the distance to a surrounding object such as a wall around the test vehicle V1, the size of the surrounding object, and the like.

Further in addition, the surrounding vehicle sensing means 30 may be an analyzer adapted to analyze the atmosphere around the test vehicle V1. The analyzer is, for example, a second vehicle-mounted exhaust gas analyzer different from the vehicle-mounted exhaust gas analyzer 10 of the above-described embodiment, and analyzes intake air flowing into the internal combustion engine E. In this case, the results of the intake air analysis (such as the concentrations of various components contained in the intake air) obtained by the second vehicle-mounted exhaust gas analyzer correspond to the surrounding vehicle information.

Such a configuration makes it possible to evaluate the contamination degree of the surrounding environment of the test vehicle V1 on the basis of the results of the intake air analysis. This may allow, for example, the test effectiveness determination part 25 to be configured to determine on the basis of the contamination degree of the surrounding environment whether exhaust gas data is effective data.

Further, by subtracting analysis results by the second vehicle-mounted exhaust gas analyzer from analysis results by the vehicle-mounted exhaust gas analyzer 10, exhaust gas analysis can be performed on the test vehicle V1 with the effect of the contamination degree of the surrounding environment eliminated.

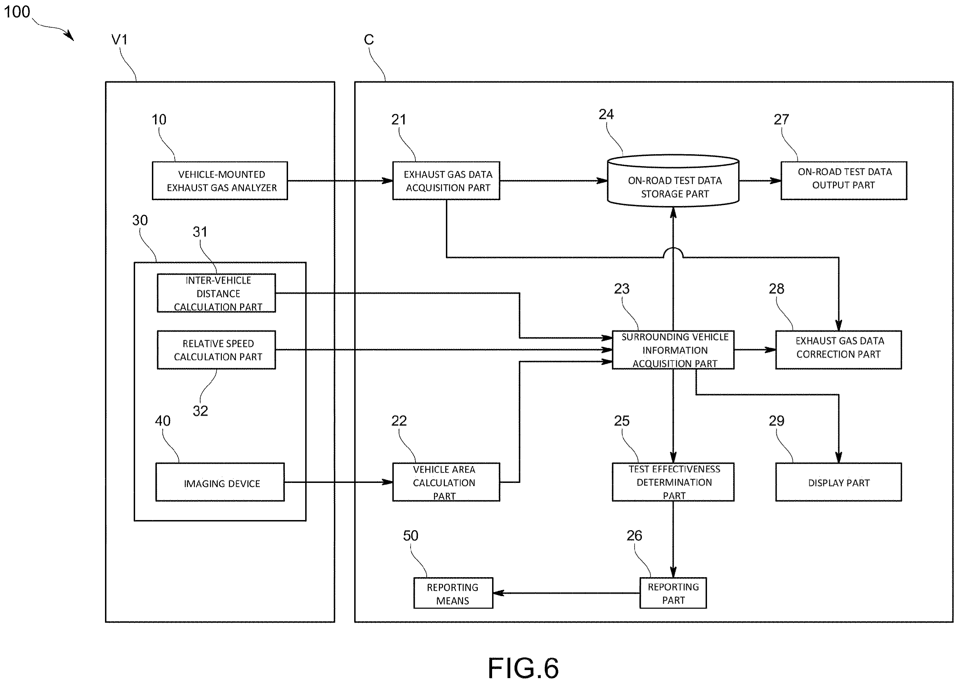

As illustrated in FIG. 6, the information processing device C may include a function as an on-road test data output part 27 adapted to comparably output exhaust gas data stored in the on-road test data storage part 24 and at least inter-vehicle distance information stored in relation to the exhaust gas data.

The on-road test data output part 27 is one adapted to display out on a display or print out on a sheet of paper analysis results indicated by the exhaust gas data and the inter-vehicle distance between the test vehicle V1 and the vehicle in front V2 during analysis indicated by the inter-vehicle distance information in the form of, for example, a graph, a table, or the like.

Describing in more detail, the on-road test data output part 27 comparably outputs exhaust gas data by the vehicle-mounted exhaust gas analyzer 10 at analysis time and an inter-vehicle distance at running time such that the analysis time and the running time coincide with each other. Note that analysis results include, for example, the concentrations of various components contained in the exhaust gas, fuel consumption, and the like. Also, the on-road test data output part 27 may be configured to comparably output, in addition to the inter-vehicle distance, the relative speed of the vehicle in front V2 and the back surface area of the vehicle in front V2 together with the analysis results. Further, the on-road test data output part 27 may comparably output the speed of the test vehicle V1, the acceleration of the test vehicle V1, and the like together with the analysis results, the inter-vehicle distance, and the like.

Such configurations make it possible to compare the analysis results and the inter-vehicle distance between the test vehicle V1 and the vehicle in front V2 during corresponding analysis, and perform the analysis, evaluation, and the like of the analysis results in consideration of the effect of the surrounding vehicle V2.

In addition, as illustrated in FIG. 6, the information processing device C may include a function as an exhaust gas data correction part 28 adapted to, on the basis of at least the inter-vehicle distance information acquired by the surrounding vehicle information acquisition part 23, correct exhaust gas data acquired by the exhaust gas data acquisition part 21.

The exhaust gas data correction part 28 calculates a correction coefficient with at least an inter-vehicle distance indicated by the inter-vehicle distance information as a parameter, and using the correction coefficient, corrects analysis results indicated by the exhaust gas data. Note that the correction coefficient may be calculated with relative speed indicated by the relative speed information, a back surface area indicated by the back surface area information, or the like as a parameter. Further, the exhaust gas data correction part 28 may be configured to calculate air resistance applied to the test vehicle V1 with at least the inter-vehicle distance indicated by the inter-vehicle distance information as a parameter, and on the basis of the air resistance, calculate the correction coefficient.

Such a configuration makes it possible to quantitatively calculate the effect of the vehicle in front V2 as the correction coefficient, and also obtain analysis results taking account of the effect.

Further, the information processing device C may include a vehicle classification part adapted to classify the vehicle in front into any of multiple types such as a light vehicle, standard-sized vehicle, and large vehicle on the basis of, for example, the back surface area information and/or image analysis. In this case, the on-road test data storage part 24 may store vehicle type information indicating the type of the vehicle in front V2 classified by the vehicle classification part and exhaust gas data acquired by the exhaust gas data acquisition part 21 in relation to each other.

Such a configuration makes it possible to perform exhaust gas analysis by the vehicle-mounted exhaust gas analyzer and the analysis, evaluation, and the like of results of the analysis in consideration of the effect of the type of the vehicle in front V2, such as a vehicle type.

In addition, the information processing device C may include a display par 29 adapted to, on the basis of the inter-vehicle distance information acquired by the surrounding information acquisition part 23, display the total or ratio of time periods during which an inter-vehicle distance indicated by the inter-vehicle distance information exceeds the predetermined threshold value. Specifically, the display part 29 displays the total time or the time ratio on a display in real time numerically, graphically, or in other such manners while the test vehicle V1 is running.

Such a configuration makes it possible to, while running, adjust the time periods during which the inter-vehicle distance exceeds the predetermined threshold value, and improve the reproducibility of exhaust gas data.

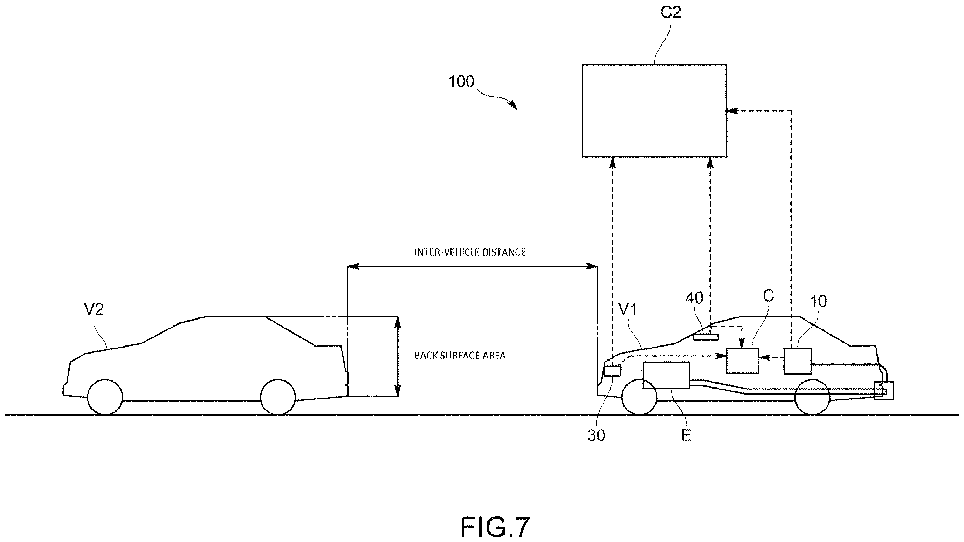

Further, regarding the functions included in the information processing device C, as illustrated in FIG. 7, part or all of them may be included in an integrated management device C2 such as a host computer provided outside the test vehicle V1 (e.g., in a test room). In this case, configurations for data transception between the integrated management device C2 and the surrounding vehicle sensing means 30 or the imaging device 40 mounted in the test vehicle V1 include one adapted to perform the data transception, for example, by wireless, via an external memory, or by other means.

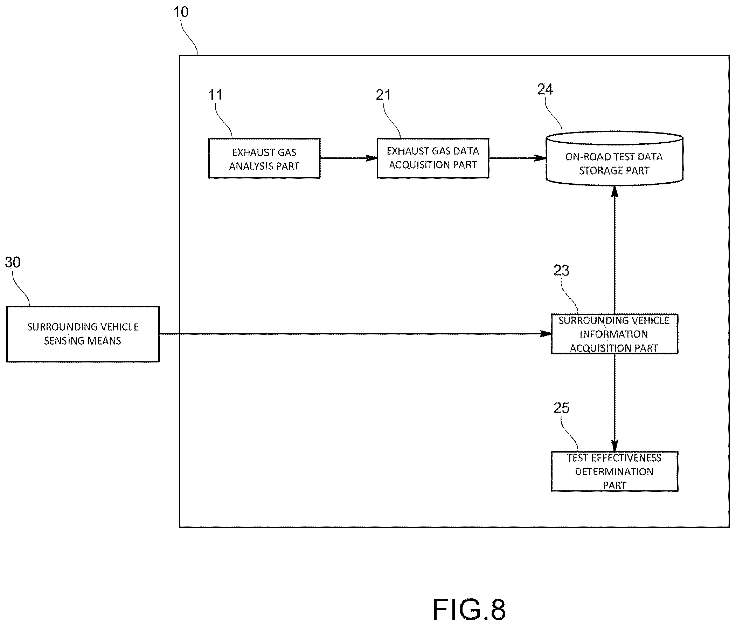

In addition, as illustrated in FIG. 8, the vehicle-mounted exhaust gas analyzer 10 may include part or all of the functions of the information processing device C in the above-described embodiment. Specifically, the vehicle-mounted exhaust gas analyzer 10 may include the exhaust gas data acquisition part 21 adapted to acquire exhaust gas data from the exhaust gas analysis part 11 while the test vehicle V1 is running and the surrounding vehicle information acquisition part 23 adapted to acquire the surrounding vehicle information from the surrounding vehicle sensing means 30.

In this case, as illustrated in FIG. 8, by further providing the vehicle-mounted exhaust gas analyzer 10 with the on-road test data storage part 24, the test effectiveness determination part 25, and the like in the above-described embodiment, it becomes possible to make the vehicle-mounted exhaust gas analyzer 10 automatically compare exhaust gas data with the surrounding vehicle information during the acquisition of the exhaust gas data, determine the effectiveness of a test taking account of the effect of the surrounding vehicle V2, or perform other such functions.

Besides, it should be appreciated that the present invention is not limited to any of the above-described embodiment and variations, but can be variously modified without departing from the scope thereof.

### Reference Signs List

100: Exhaust gas analysis system
V1: Test vehicle
V2: Surrounding vehicle (vehicle in front)
10: Vehicle-mounted exhaust gas analyzer
21: Exhaust gas data acquisition part
30^{:} Surrounding vehicle sensing means
31: Inter-vehicle distance calculation part
32: Relative speed calculation part
40: Imaging device
23: Surrounding vehicle information acquisition part
24: On-road test data storage part

## Claims

1. A vehicle-mounted exhaust gas analyzer mounted in a test vehicle running on a road, the vehicle-mounted exhaust gas analyzer comprising:
an exhaust gas data acquisition part adapted to acquire exhaust gas data on exhaust gas discharged from the test vehicle during a run of the test vehicle; and
a surrounding vehicle information acquisition part adapted to, from surrounding vehicle sensing means mounted in the test vehicle, acquire surrounding vehicle information that is information on a vehicle running around the test vehicle.

2. The vehicle-mounted exhaust gas analyzer according to claim 1, comprising
an on-road test data storage part adapted to store the exhaust gas data and the surrounding vehicle information in relation to each other.

3. The vehicle-mounted exhaust gas analyzer according to claim 1 or 2, wherein
the surrounding vehicle information includes inter-vehicle distance information indicating an inter-vehicle distance between the test vehicle and the surrounding vehicle during the acquisition of the exhaust gas data by the exhaust gas data acquisition part.

4. The vehicle-mounted exhaust gas analyzer according to any one of claims 1 to 3, wherein
the surrounding information is an analysis result of analyzing an atmosphere around the test vehicle.

5. The vehicle-mounted exhaust gas analyzer according to any one of claims 1 to 4, wherein
at least one of a millimeter wave radar or an imaging device is mounted in the test vehicle as the surrounding vehicle sensing means.

6. The vehicle-mounted exhaust gas analyzer according to claim 5, wherein
the surrounding vehicle information acquisition part acquires vehicle area information indicating a back surface area of a vehicle in front with use of imaged data by the imaging device, the back surface area being imaged during exhaust gas analysis.

7. The vehicle-mounted exhaust gas analyzer according to any one of claims 3 to 6, comprising
a test effectiveness determination part adapted to, on a basis of at least the inter-vehicle distance information, determine effectiveness of the exhaust gas data in an on-road running test of the test vehicle.

8. The vehicle-mounted exhaust gas analyzer according to calm 7, wherein
the test effectiveness determination part measures time periods during which the inter-vehicle distance indicated by the inter-vehicle distance information exceeds a predetermined threshold value, and when the time periods exceed a predetermined time period continuously or in total, determines that the exhaust gas data is effective data.

9. The vehicle-mounted exhaust gas analyzer according to claim 7 or 8, comprising
a reporting part adapted to, when the test effectiveness determination part determines that the exhaust gas data is effective data, report a result of the determination during the exhaust gas analysis.

10. The vehicle-mounted exhaust gas analyzer according to any one of claims 3 to 9, comprising
a display part adapted to display a total of the time periods or distances during which the inter-vehicle distance indicated by the inter-vehicle distance information exceeds the predetermined threshold value, or a ratio of the total of the time periods or the distances to a total run period or distance of the test vehicle.

11. An information processing device used together with a vehicle-mounted exhaust gas analyzer mounted in a test vehicle running on a road, the information processing device comprising:
an exhaust gas data acquisition part adapted to, from the vehicle-mounted exhaust gas analyzer, acquire exhaust gas data on exhaust gas discharged from the test vehicle during a run of the test vehicle; and
a surrounding vehicle information acquisition part adapted to, from surrounding vehicle sensing means mounted in the test vehicle, acquire surrounding vehicle information that is information on a vehicle running around the test vehicle.

12. An exhaust gas analysis system comprising:
the vehicle-mounted exhaust gas analyzer; and
the information processing device according to claim 11.

13. A program used for a vehicle-mounted exhaust gas analyzer mounted in a test vehicle running on a road, the program instructing a computer to fulfill functions as:
an exhaust gas data acquisition part adapted to acquire exhaust gas data on exhaust gas discharged from the test vehicle during a run of the test vehicle; and
a surrounding vehicle information acquisition part adapted to, from surrounding vehicle sensing means mounted in the test vehicle, acquire surrounding vehicle information that is information on a vehicle running around the test vehicle.

14. An exhaust gas analysis method comprising steps of:
acquiring exhaust gas data on exhaust gas discharged from the test vehicle during a run of the test vehicle with use of a vehicle-mounted exhaust gas analyzer mounted in a test vehicle; and
acquiring surrounding vehicle information that is information on a vehicle running around the test vehicle from surrounding vehicle sensing means mounted in the test vehicle.
